# NEUE EUROPÄISCHE PATENTSCHRIFT

(11) **EP 1 006 148 B2**
(45) Veröffentlichungstag und Bekanntmachung des Hinweises auf die Entscheidung über den Einspruch: **30.08.2006**
(45) Hinweis auf die Patenterteilung: 15.10.2003
(21) Anmeldenummer: 99114125.0
(22) Anmeldetag: 19.07.1999
(51) Int. Cl.: C08L 1/02, A61K 9/20, A61K 47/38, A61K 47/36

(54) **Coprozessiertes Polysaccharidprodukt**
Coprocessed polysaccharide product
Produit polysaccharidique co-travaillé

(30) Priorität: 30.11.1998 DE 19855203
(43) Veröffentlichungstag der Anmeldung: 07.06.2000
(73) Patentinhaber: fit GmbH, 02788 Hirschfelde (DE)
(72) Erfinder: Bauer, Kurt H., Prof. Dr., 79112 Freiburg (DE)
(74) Vertreter: Sommer, Peter

(56) Entgegenhaltungen:
- WO-A-87/05804
- WO-A-98/03064
- GB-A- 1 146 352
- US-A- 4 097 606
- US-A- 5 834 021
- Pharmazeutische Technologie, G.Fischer Verlag, Stuttgart, Jena, 5. Auflage, 1997, Seiten 331-332

## Beschreibung

Die Erfindung betrifft neue mit Natrium-Carboxymethylstärke coprozessierte Celluloseprodukte, die sich durch eine außerordentlich schnelle und starke Quellung beim Einbringen in Wasser oder wasserhaltige Flüssigkeiten auszeichnen. Die schnelle und starke Quellung wird durch eine möglichst hohe bzw. optimale Verdichtung des Zweikomponentensystems bei einem trockenen oder feuchten Coprozeß noch erheblich verbessert. Die neuen Produkte sind auch durch eine sehr gute Tablettierbarkeit charakterisiert. Sie können daher mit Erfolg als Tablettensprengmittel (Zerfallsbeschleuniger, Zerfallshilfsmittel) eingesetzt werden.

Als Tablettensprengmittel (Zertallsbeschleuniger, Zerfallhilfsmittel) werden Hilfsstoffe bezeichnet, die für den raschen Zerfall von Tabletten in Wasser oder Magensaft und für die Freisetzung der Pharmaka in fein dispergierter, gut resorbierbarer Form sorgen. Je nach Wirkungsmechanismus handelt es sich um Substanzen, die die Porosität der Komprimate erhöhen und ein großes Adsorptionsvermögen für Wasser besitzen. Beispiele für bekannte Tablettensprengmittel sind Stärke, Cellulosederivate, Alginsäuren, Dextrane und quervernetzte Polyvinylpyrrolidone sowie gasentwickelnde Substanzgemische, z.B. Natriumhydrogencarbonat und Zitronen-oder Welnsäure (Brausemischungen).

Die im Stand der Technik bekannten Tablettensprengmittel auf Basis von Stärke und Cellulosederivaten sind aber noch mit folgenden Nachteilen behaftet:

Stärkekörner allein sind elastisch und deshalb schlecht plastisch verformbar bzw. tablettierbar und deshalb bessere Zerfallsbeschleuniger. Celluloseprodukte sind dagegen mehr plastisch und deshalb gut tablettierbar. Cellulosetabletten quellen auch sehr gut, ohne jedoch dabei zu zerfallen. Mischungen aus diesen beiden Substanzklassen vereinigen die Vorteile der guten Tablettierbarkeit, der starken Quellung bzw. der schnellen Zerfallbarkeit.

Aus der WO 87/50804 A1 ist ein Verfahren zur Herstellung eines Polysaccharidprodukts bekannt, das unter anderem Natrium-Carboxymethylstärke und mikrokristalline Cellulose aufweist, welche zusammen mit anderen Rohstoffen durch Nassgranulierung der Gesamtmischung in ein Granulat überführt werden. Dabei kann die Natrium-Carboxymethylstärke auch mit mikrokristalliner Cellulose vermischt der restlichen Rohstoffmischung beigegeben werden, wobei jedoch nach wie vor die Nassgranulierung zusammen mit den anderen Rohstoffen zu einem Fertiggranulat für die Tablettierung erfolgt.

Der Erfindung liegt die Aufgabe zugrunde, die bekannten Tablettensprengmittel auf der Basis von Stärke und/ oder Cellulosederivaten zu verbessern und insbesondere Tablettensprengmittelprodukte bereitzustellen, die beim Einbringen in Wasser sofort, also in Sekundenschnelle, quellen, zerfallen und sich dispergieren. Daneben sollen je nach dem Vermahlungsgrad instantartig gröbere, feine und feinste bis kolloidartige Dispersionen herstellbar sein. Weiterhin sollen erfindungsgemäß Dispersions- bzw. Suspensionsstabilisatoren bereitgestellt werden, die mit Vorteil bei der Herstellung von Pharmazeutika, Kosmetika, Nahrungsmitteln und dergleichen, die sich sehr rasch dispergieren sollen, verwendet werden können.

Diese Aufgabe wird erfindungsgemäß durch ein Polysaccharidprodukt, umfassend Natrium-Carboxymethylstärke und pulverisierte oder mikrokristalline Cellulose (MCC), gelöst.

Das erfindungsgemäße Polysaccharidprodukt wird durch Coprozessierung bzw. gemeinsame Behandlung von Natrium-Carboxymethylstärke mit pulverisierter oder mikrokristalliner Cellulose durch feuchte oder trockene Aggreglerung unter Druck Zusammengebracht und verdichtet. Unter dem Begriff "Coprozessierung" wird hier eine trokkene Kompaktierung/Aggregierung, z.B. zwischen gegenläufigen Kompaktierwalzen bei Drücken von 20-60 kN, vorzugsweise 30-50 kN, oder eine feuchte Kompaktierung/Aggregierung nach Zusatz von Wasser, durch Kneten oder Pressen von feuchtplastischen Massen durch ein Sieb, eine Lochscheibe oder über einen Extruder und abschließendem Trocknen verstanden.

Die erfindungsgemäße, durch Coprozessierung von Natrium-Carboxymethylstärke mit pulverisierter oder mikrokristalliner Cellulose (MCC) erhaltene Polysaccharidprodukt ist durch folgende Kenndaten charakterisiert:

Bei den gemahlenen, coprozessierten Zubereitungen sollen die Schüttdichten der Produkte mit Cellulosepulverbei 0,120-0,500 g/ml liegen, vorzugsweise bei 0,200-0,400 g/ml. Bei den Coprodukten mit mikrokristalliner Cellulose (MCC) und Natrium-Carboxymethylstärke liegen die Schütteldichten bei 0,300-0,750 g/ml, vorzugsweise bei 0,350-0,600 g/ml. Die Schüttdichte des erfindungsgemäßen Polysaccharid-Produkts ist vorzugsweise höher als die Schüttdichte des Ausgangspulvergemisches.

Insbesondere ist das Produkt durch einen Restfeuchtegehalt von 1,5 bis 15%, vorzugsweise von weniger als 10%, beispielsweise 4 bis 7%, charakterisiert.

Der Gehalt der einzelnen Komponenten kann innerhalb weiter Grenzen variieren, beispielsweise von 1 bis 60 Gew.% Natrium-Carboxymethylstärke und 40 bis 99 Gew.-% pulverisierter oder mikrokristalliner Cellulose. Bevorzugt wird ein Gehalt von 3 bis 60 Gew.% Natrium-Carboxymethylstärke und 40 bis 97 Gew.-% pulverisierter oder mikrokristalliner Cellulose. Ganz bevorzugt werden Mengen von 5 bis 30 Gew.% Natrium-Carboxymethylstärke und 70 bis 95 Gew.% pulverisierter oder mikrokristalliner Cellulose.

Auch die Korngröße des erfindungsgemäßen Polysaccharidprodukts kann innerhalb weiter Grenzen variieren, wie beispielsweise von 10 bis 1000 µm, vorzugsweise 50 bis 500 µm.

Durch die Erfindung wird weiterhin ein Verfahren zur Herstellung des oben beschriebenen Polysaccharidprodukts bereitgestellt, welches die Coprozessierung von hochmolekularer unlöslicher Kartoffel- oder Mais-Carboxymethylstärke in Form des Natrium Salzes mit mikrokristalliner oder pulverisierter Cellulose umfaßt.

Eine Alternative des erfindungsgemäßen Verfahrens ist durch folgende Stufen charakterisiert:
(a) Suspendieren von feuchter bzw. nicht getrockneter, nicht verhornter mikrokristalliner oder pulverisierter Cellulose in einem, vorzugsweise heißen, wäßrigen Medium zu einer homogenen Dispersion;
(b) Vermengen der in Stufe (a) erhaltenen Suspension oder pastenförmigen vorverdichteten Massen nach Abkühlen unter 40°C mit hochmolekularer unlöslicher Kartoffel- oder Mais-Carboxymethylstärke in Form des Natrium Salzes;
(c) gegebenenfalls erfolgendes Vortrocknen;
(d) Aggregieren unter Druck, Insbesondere verdichtendes Passieren, vorzugsweise Pressen der in Stufe (b) bzw.
(c) erhaltenen und vorverdichteten feuchtplastischen Masse durch ein Sieb, eine Lochscheibe oder einen Extruder;
(e) Trocknen der in Stufe (d) erhaltenen aggregierten Masse bei einer Temperatur von 20 bis 90°C; und
(f) Vermahlen des so erhaltenen Produkts auf eine mittlere Korngröße von 10 bis 1000 µm.

In der ersten Stufe dieses Verfahrens wird feuchte, nicht verhomte pulverförmige oder mikrokristailine Cellulose in einem wäßrigen Medium zu einer homogenen Dispersion suspendiert.

Als Cellulose-Ausgangsprodukt wird vorzugsweise ein nicht getrocknetes mikrokristallines Celluloseprodukt oder pulverisierte Cellulose verwendet. Die pulverförmige Cellulose wird beispielsweise in an sich bekannter Weise aus Zellstoff oder faseriger Rohcellulose hergestellt. Die mikrokristalline Cellulose wird aus Zellstoffpulpe durch Erhitzen mit einer 3- bis 5%-igen starken Mineralsäure, beispielsweise Salz- oder Schwefelsäure, hergestellt. Für die Zwekke der Erfindung besonders geeignete, nicht getrockneten Celluloseprodukte sind praktisch Zwischen- oder Vorprodukte der Herstellung von reiner Pulvercellulose oder mikrokristalliner Cellulose.

Die oben erwähnte Herstellung von mikrokristalliner Cellulose aus Zellstoff oder von Pulvercellulose wird beispielsweise von B. Philipp und H.H. Steege in "Wissenschaft und Fortschritt"' 25 (3), 126-131 (1975) beschrieben.

Unter "nicht getrocknet" werden Cellulosevorprodukte verstanden, die aus dem Herstellungsgang, nach der Reinigung, aber vor der Endtrocknung gewonnen werden, z.B. bei der oben beschriebenen Behandlung von Zellstoffpulpe mit Mineralsäure nach Entfernen der Säure und der erforderlichen Reinigung der hierbei entstandenen mikrokristallinen Cellulose. Das Cellulose:Wasser-Verhältnis kann 1,0:0,15 Teile bis 1,0:30,0 Teile, vorzugsweise 1,0:1,0 Teile bis 1,0:10,0 Teile betragen. Bei einer Trocknung des Produkts zu einem Feuchtigkeitsgehalt von weniger als 1,5 Teile Wasser pro 10,0 Teile Cellulose kann es vorkommen, daß die Oberfläche der Cellulose zu trocken wird und daß das Produkt in unerwünschter Weise "verhornt". In solchen Fällen sind die Polysaccharidprodukte nicht mehr optimal fürdie oben angegebenen Zwecke geeignet, da verhomte Bereiche nurmehr stark eingeschränkt hydratisieren können. Andererseits führen zu große Flüssigkeits- bzw. Wassermengen dazu, daß die Produkte nach der Zugabe von Natrium-Carboxymethylstärke nicht ausreichend feuchtplastisch werden, sondern zu flüssig sind, so daß eine Obergrenze von 30,0 Teile, vorzugsweise 25,0 Teile Wasser pro 1,0 Teil Cellulose bevorzugt wird. Außerdem ist das Trocknen von hohen Feuchtigkeitsanteilen nicht wirtschaftlich. Relativ hohe Mengen von Wasser sind zu einer ausreichenden Verdichtung erforderlich, aber zu hohe Mengen können entsprechend den in Stufe (b) verwendeten Mengen der Natrium-Carboxymethylstärke und dem Polymerisationsgrad dieses Stärkeprodukts die Gemische dann zu feucht werden lassen, so daß sie nicht mehrfeuchtplastisch verformbar bzw. aggregierbar sind und nur noch langwierig unter Schwierigkeiten getrocknet werden können.

Somit wird in der ersten Stufe dieses Verfahrens die oben beschriebene nicht verhomte Cellulose in einem wäßrigen, zur besseren Hydratisierung vorzugsweise warmen bis heißen Medium zu einer homogenen Dispersion suspendiert. Als wäßriges Medium kann beispielsweise Wasser, wie Leitungswasser, verwendet werden, das gegebenenfalls bis zum Sieden aufgeheizt werden kann. Die Temperatur beim Suspendierungsvorgang beträgt 40 bis 100°C, vorzugsweise 80 bis 100°C. Die Suspendierung kann in an sich bekannter Weise in einer geeigneten Vorrichtung, wie einem Schnellmischer oder Dispergator, verwendet werden. Geeignete Vorrichtungen sind im Handel erhältlich und werden beispielsweise in Bauer, Frömming, Führer: Pharmazeutische Technologie, G. Fischer Verlag, Stuttgart, Jena, 5. Auflage, 1997, Seite 135 beschrieben. Bei dieser Befeuchtung, d.h. bei diesem Suspensionsvorgang, kann gegebenenfalls schon eine Vorverdichtung erzielt werden, z.B. durch stärkere Hydratation bei Verwendung von heißem Wasser. Weiterhin kann durch Kneten, beispielsweise im Z-Kneter mit kräftigen Mischarmen, im Gegensatz zu einfach gerührten bzw. gemischten Massen eine stärkere Verdichtung oder Vorverdichtung erreicht werden. Die so erhaltene Dispersion hat beispielsweise einen Feststoffgehalt von 10 bis 90 Gew.-%, vorzugsweise 20 bis 60 Gew.-%.

In der nächsten Stufe (b) dieses Verfahrens wird die in Stufe (a) erhaltene Suspension nach Abkühlung unter 40°C mit Natrium-Carboxymethylstärke vermengt. Hierbei können alle beliebigen im Handel erhältlichen Natrium-Carboxymethylstärkesorten verwendet werden. Geeignete hochmolekularer unlöslicher Kartoffel- oder Mais-Carboxymethylstärke in Form des Natrium Salzes werden beispielsweise in den Firmenprospekten über Natrium-Carboxymethylstärke der Firmen CHP Carbohydrate Pirna GmbH, D-01796 Pirna, und J. Rettenmaier & Söhne GmbH + Co., D-73494 Rosenberg, beschrieben.

Bevorzugte Carboxymethylstärkeprodukte sind handelsübliche hochmolekulare, unlösliche Kartoffel- oder Mais-Carboxymethylstärken in Form des Natrium salzes.

Die Menge der in dieser Stufe zugegebenen Natrium-Carboxymethylstärke wird so bemessen, daß die als Endprodukt erhaltenen Polysaccharidprodukte 3,0 bis 60 Gew.%, vorzugsweise 5 bis 40 Gew.%, Natrium-Carboxymethylstärke enthalten.

Die Vermengung der beiden Ausgangsprodukte kann vorzugsweise durch Verkneten geschehen. Geeignete Kneterwerden beispielsweise in Bauer, Frömming, Führer: Pharmazeutische Technologie, G. Fischer Verlag, Stuttgart, Jena, 5. Auflage, 1997, Seite 134-135 beschrieben. Die Temperatur bei der Vermengung bzw. Verknetung beträgt 50 bis 30°C, vorzugsweise 40 bis 20°C. Vorzugsweise erfolgt die Verknetung bei Raumtemperatur, damit das Stärkeprodukt nicht nachteilig beeinflußt wird. Eine Verknetung kann daneben auch grundsätzlich dann vorgenommen werden, um eine Verdichtung zu erzielen.

In der nächsten Stufe (c) dieses Verfahrens wird das in Stufe (b) erhaltene Gemisch gegebenenfalls zu einem Restfeuchtegehalt von 1,5 bis 15 Gew.%, vorzugsweise 4 bis 7 Gew.%, vorgetrocknet. Als Trockner können übliche Trocknungsvorrichtungen, wie Trockenschränke und Wirbelschichttrockner eingesetzt werden. Geeignete Trockner werden beispielsweise in Bauer, Frömming, Führer: Pharmazeutische Technologie, G. Fischer Verlag, Stuttgart, Jena, 5. Auflage, 1997, Seiten 123-130 beschrieben. Bevorzugt wird die Vortrocknung bei Temperaturen von 30 bis 70°C, insbesondere 50 bis 60°C durchgeführt.

In der nächsten Stufe (d) dieses Verfahrens wird das in Stufe (b) bzw. (c) erhaltene feuchtplastische, durch das Befeuchten bereits vorverdichtete Produkt durch ein Sieb geschlagen bzw. gepreßt und dadurch möglichst weitgehend verdichtet. Als Siebe kommen beispielsweise übliche Siebe, die beispielsweise in Bauer, Frömming, Führer: Pharmazeutische Technologie, G. Fischer Verlag, Stuttgart, Jena, 5. Auflage, 1997, Seite 108 (Siebe), Seite 308 (Extrudergranulierung) beschrieben werden, in Betracht. Geeignete Siebe haben eine Maschenweite von 0,5 mm bis 5 mm, vorzugsweise 1-2 mm. Im übrigen erfolgt das Durchpressen bzw. -schlagen des Produkts durch Siebe bzw. durch Extruder in üblicher Weise und bei üblichen Bedingungen, wie Normaltemperatur. Das Sieben erfolgt bei Normaldruck oder gegebenenfalls unter Anwendung von Überdruck.

In der nächsten Stufe (e) dieses erfindungsgemäßen Verfahrens wird die durch das Sieb geleitete Masse bei einer Temperatur von 20 bis 90°C, vorzugsweise 50 bis 80°C getrocknet. Die Trocknung erfolgt In der gleichen Weise wie die oben im Zusammenhang mit Stufe (c) beschriebene Vortrocknung. Auch hier können übliche Trockner, wie Trockenschränke und Wirbelschichttrockner, eingesetzt werden.

In der letzten Stufe (f) dieses Verfahrens wird schließlich das so erhaltene Produkt auf eine mittlere Komgröße von 10 bis 1000 µm, vorzugsweise 50 bis 500 µm, vermahlt. Für den Vermahlungsvorgang können alle beliebigen geeigneten Mühlen, wie beispielsweise Kugelmühlen und dergleichen, verwendet werden. Geeignete Vorrichtungen sind beispielsweise in Bauer, Frömming, Führer: Pharmazeutische Technologie, G. Fischer Verlag, Stuttgart, Jena, 5. Auflage, 1997, Seite 104-107 beschrieben.

Eine andere Alternative des erfindungsgemäßen Verfahrens ist durch folgende Stufen charakterisiert:
(a) Vermischen der pulverförmigen Bestandteile hochmolekularer unlöslicher Kartoffel- oder Mais-Carboxymethylstärke in Form des Natrium Salzes und Cellulose;
(b) Sieben durch ein feines Sieb;
(c) Verdichten durch Kompaktieren oder Brikettieren mit Kompaktierwalzen oder einer Exzenterpresse bei Drücken von 20-60 kN, vorzugsweise 30-50 kN;
(d) Granulieren bzw. verkleinerndes Homogenisieren der nach (c) erhaltenen Briketts oder Schülpen mit Stachelwalzen oder einem ähnlichen Gerät oder Vermahlen mit einer geeigneten Mühle auf Komgrößen von 10-1000 µm; und
(e) Vermahlen des so erhaltenen Produkts auf eine mittlere Korngröße von 10 bis 1000 µm.

Bei dieser Alternative entfallen die Herstellungsschritte der Suspendierung (a), des Vortrocknens (c), das Pressen durch Siebe oder Extruder (d) und der Endtrocknung (e) der ersten Alternative. Es verbleiben nur die Schritte Vermischen der beiden Komponenten (a), einfaches Sieben (b), zum Beispiel durch ein Sieb mit einer Maschenweite von 1,2 mm, und als Hauptschritt die Kompaktierung mit Druck- bzw. Kalanderwalzen oder Exzenterpressen (c), die nachfolgende Zerkleinerung der kompaktierten Schülpen oder Briketts mit Stachelwalzen (d) und das Vermahlen mit geeigneten Mühlen (e).

Die Qualität der Verdichtung hängt von den Kompaktierdrucken (20-60 kN, vorzugsweise 30-50 kN) und in zweiter Linie auch noch von den Restfeuchtigkeitsgehalten der Mischung (b) ab, die zwischen 3 und 40 Gew.-%, vorzugsweise zwischen 5 und 25 Gew.% liegen kann. Endprodukte mit den höheren Feuchtigkeitsgehalten müssen evtl. nachgetrocknet werden, bis sie Restfeuchtigkeiten von 1,5-15 Gew.%, vorzugsweise von 4-7 Gew.% aufweisen. Die Trockenverdichtungs- bzw. Trockengranulierverfahren werden beispielsweise in Bauer, Frömming, Führer: Pharmazeutische Technologie, G. Fischer Verlag, Stuttgart, Jena, 5. Auflage, 1997, Seite 306, 308 und 310 beschrieben.

Die so hergestellten erfindungsgemäßen, mit Natrium-Carboxymethylstärke coprozessierten Celluloseprodukte zeichnen sich durch eine außerordentlich schnelle und starke Quellung beim Einbringen in Wasser oder wasserhaltige Flüssigkeiten sowie durch eine sehr gute Tablettierbarkeit aus. Sie können daher als äußerst wirksame Tablettensprengmittel (Zerfallhilfsmittel bzw. Zerfallsbeschleuniger) eingesetzt werden.

Unter Verwendung der erfindungsgemäßen Produkte können Dispersionstabletten hergestellt werden, die beim Einbringen in Wasser sofort, also in Sekundenschnelle, quellen, zerfallen und sich dispergieren. Je nach Vermahlungsgrad des erfindungsgemäßen Produkts lassen sich aus den damit hergestellten Tabletten instantartig gröbere, feine und feinste bis kolloidartige Dispersionen erzeugen. Neben der Verwendbarkeit als Tablettenzerfallsbeschleuniger ist natürlich ein Einsatz als Dispersionsoder Suspensionsstabilisator bei der direkten Herstellung von flüssigen und halbfesten Arzneizubereitungen wie Sirupen, Lotionen, Säften, Gelen, Hydrosolen und Hydrogelen, Salbenzubereitungen und Pasten denkbar und mögüch. Die Einsatzmöglichkeiten sind überall möglich, wo schnelle und feinste Dispergierungen erreicht und stabilisiert werden sollen. Die Anwendung ist nicht auf Pharmazeutika und Kosmetika beschränkt. Es drängen sich auch Verwendungen bei nahrungsmitteltechnologischen und technischen Aufgabenstellungen auf. Beispielsweise soll hier die Stabilisierung von Speiseeis oder Softeis, die Verdickung und Dispergierung von Soßen genannt werden, sowie auch Instantzubereitungen, wie Kakao- und andere Mixgetränke, die aus pulver- oder granulatartigen Vorprodukten augenblicklich anrührbar oder dispergierbar sein sollen.

Die Erfindung wird in den Beispielen erläutert.

### 1. Herstellungsbeispiele

### Beispiel 1:

1000,0 kg feuchtes, nicht verhomtes Cellulosepulver mit einem Wassergehaft von ca. 20% wird möglichst bald nach der Herstellung durch Zugabe von 2500,0 kg Wasser resuspendiert. Die Suspension wird mit einem Schnellmischer oder Dispergator homogen dispergiert, und anschließend werden 160,0 kg handelsüblicher Natrium-Carboxymethylstärke aus Kartoffelstärke eingeknetet. Die feuchtplastische Masse wird durch einen Extruder mit 1 mm Maschenweite geschlagen und in einem geeigneten Trockner bei 60°C getrocknet.

### Beispiel 2:

2250,0 kg feuchter Kuchen mit einem Verhältnis mikrokristalline Cellulose:Wasser= 40:60 wird mit der gleichen Menge Wasser resuspendiert und homogenisiert. Anschließend werden 100,0 kg Carboxymethylstärke-Natrium aus Maisstärke gleichmäßig eingeknetet, die feuchtplastische Masse durch ein 1,5 mm Sieb geschlagen und bei 80°C in einem Wirbelschichttrockner bis zu einer Restfeuchte von ca. 5% getrocknet.

### Beispiel 3:

2000,0 kg feuchter Kuchen aus mikrokristalliner Cellulose und Wasser im Verhältnis 50:50 wird mit der doppelten Menge Wasser angeschlämmt bzw. resuspendiert und 5 min lang mit dem Ultra-Turrax homogenisiert.

Anschließend wird die homogene Mischung in einen Planetenmischen gegeben, und bei laufendem Rührwerk wird portionsweise 300,0 kg Natrium-Carboxymethylstärke dazugeknetet und abschließend 10 min gemischt.

Die feuchtplastische Masse wird -in dünnen Schichten und angemessenen Portionen entweder im Trockenschrank bei 50°C oder im Wirbelschicht-Trockner bei einer Zulufttemperatur von 70°C bis zur siebbaren Konsistenz vorgetrocknet. Dann wird sie durch ein 5mm-Sieb geschlagen und im Wirbelschicht-Trockner bei einerZulufttemperatur von 70° zu Ende getrocknet (Restfeuchte ca. 5%).

Zuletzt wird die Masse durch ein 1,00 mm-Sieb geschlagen und in einer geeigneten Mühle auf eine mittlere Komgröße von 80 µm gemahlen.

### Beispiel 4:

| | |
|---|---|
| 2000,0 g | feuchtes, feingemahlenes Cellulosepulver (mittlere Partikelgröße 50 µm, Feuchtigkeitsgehalt ca. 50%) wird mit |
| 1500,0 g | siedendem, deionisiertem Wasser in einem geeigneten Mischer homogen befeuchtet. Anschließend wer-den |
| 100,0 g | Natrium-Carboxymethylstärke eingeknetet, so daß eine feuchtplastische Masse entstanden ist. Diefeuchtplastische Masse wird mit möglichst hohem Druck durch einen Extruder mit einer Lochscheibe mit 1-3 mm-Bohrungen (vorzugsweise 1-2 mm) gepreßt. Das entstandene Extrudergranulat wird bei 80°C bis zu einer Restfeuchte von 4-6% getrocknet und anschließend mit einer Rotor-Stator- oder Stift-Mühle auf eine mittlere Korngröße von 0,2 mm vermahlen. |

### Beispiel 5:

| | |
|---|---|
| 9,0 kg | feingemahlenes Cellulosepulver (mittlere Partikelgröße 40 µm) und |
| 1,0 kg | Natrium-Carboxymethylstärke werden homogen gemischt und durch ein 1 mm-Sieb geschlagen. Anschließend wird diese Mischung mit Hilfe einer Kompaktierwalze bei Drucken von 30-45 kN brikettiert und durch ein 0,2 mm- oder 1 mm-Sieb geschlagen. Die Schütteldichte liegt bei 0,400-0,450 g/ml. |

### 2. Verwendungsbeispiel

Unter Verwendung der erfindungsgemäßen, mit Carboxymethylstärke coprozessierten gemahlenen Cellulosewurden Tabletten in folgender Weise hergestellt:

| | |
|---|---|
| ASS (Acetylsalicylsäure), mittlere Korngröße 0,2 mm | 1000,0 |
| Cellulose-Carboxymethylstärke Coprocessed | 100,0 |

werden gemischt und auf einer üblichen Tablettenpresse zu Tabletten mit 11 mm Durchmesser und 550 mg Bruttogewicht verpreßt.

Die im Verwendungsbeispiel hergestellten Tabletten wurden folgendem Zerfallstest in folgender Weise unterworfen:
a) einfaches Einlegen der Tabletten in ein Becherglas mit ca. 200-250 ml reines Wasser bei Raumtemperatur und Beobachtung des Zerfalls;
b) Einbringen jeweils einer Tablette, die in einem Drahtbügel eingeklemmt ist, in ein Becherglas mit 250 ml reinem Wasser bei Raumtemperatur und Bestimmung des Zeitraums bis zum vollständigen Zerfall.

Die erhaltenen Ergebnisse sind in folgender Tabelle zusammengestellt:

| | a) | b) |
|---|---|---|
| Tabl. mit 500 mg ASS + 50 mg Cellulose coprocessed nach Beispiel 1: | zerfällt sehr rasch | 10-15 s |
| Tabl. mit 500 mg ASS + 50 mg Cellulose coprocessed nach Beispiel 2: | " | 20-25 s |
| Tabl. mit 500 mg ASS+50 mg Cellulose coprocessed nach Beispiel 5: | " | 16-17 s |
| Tabl. mit 500 mg ASS + 50 mg Weizenstärke: | quillt stark, aber zerfällt nicht | 35-45 s |

Aus der obigen Tabelle ergibt sich, daß die unter Verwendung der erfindungsgemäßen Produkte hergestellten Tabletten im Vergleich zu den Produkten mit herkömmlichen Sprengmitteln wesentlich verbesserte Zerfallseigenschaften haben.

## Patentansprüche

1. Polysaccharidprodukt, bestehend aus hochmolekularer unlöslicher Kartoffel- oder Mais-Carboxymethylstärke in Form des Natrium-Salzes und mikrokristalliner Cellulose oder pulverisierter Cellulose, **dadurch gekennzeichnet, dass** die unlösliche Natrium-Carboxymethylstärke und die mikrokristalline Cellulose oder pulverisierte Cellulose durch Coprozessierung mittels feuchter oder trockener Aggregierung unter Druck zusammengebracht und verdichtet sind.

2. Polysaccharidprodukt nach Anspruch 1, **dadurch gekennzeichnet, dass** das Polysaccharidprodukt gegenüber dem Ausgangspulvergemisch eine höhere Schüttdichte besitzt.

3. Polysaccharidprodukt nach Anspruch 1 oder 2, **dadurch gekennzeichnet, dass** es eine Restfeuchte von weniger als 10% besitzt.

4. Polysaccharidprodukt nach einem der Ansprüche 1 bis 3, **dadurch gekennzeichnet, dass** es 3 bis 60 Gew.% unlösliche Natrium-Carboxymethylstärke und 40 bis 97 Gew.-% mikrokristalline oder pulverisierte Cellulose enthält.

5. Polysaccharidprodukt nach einem der Ansprüche 1 bis 4, **dadurch gekennzeichnet, dass** es eine mittlere Komgröße von 10 bis 1000 µm hat.

6. Verfahren zur Herstellung eines aus hochmolekularer unlöslicher Kartoffel- oder Mais-Carboxymethylstärke in Form des Natrium-Salzes und mikrokristalliner oder pulverisierter Cellulose bestehenden Polysaccharidprodukts, umfassend eine Caprozessierung durch feuchte oder trockene Aggregierung unter Druck von unlöslicher Natrium-Carboxymethylstärke mit mikrokristalliner Cellulose oder pulverisierter Cellulose einhergehend mit einer Verdichtung.

7. Verfahren nach Anspruch 6, **dadurch gekennzeichnet, dass** es die folgenden Stufen umfasst:
a) Suspendieren und gegebenenfalls Verdichten von feuchter bzw. nicht getrockneter, nicht verhornter mikrokristalliner oder pulverisierter Cellulose In einem wässrigen Medium zu einer homogenen Dispersion;
b) Vermengen der in Stufe a) erhaltenen Suspension oder pastenförmigen, vorverdichteten Massen nach Abkühlen unter 40° C mit Natrium-Carboxymethylstärke;
c) gegebenenfalls erfolgendes Vortrocknen;
d) Verdichtendes Passieren, vorzugsweise Pressen der in Stufe b) bzw. c) erhaltenen und vorverdichteten feuchtplastischen Masse durch ein Sieb, eine Lochscheibe oder einen Extruder;
e) Trocknen der In Stufe d) erhaltenen aggregierten Masse bei einer Temperatur von 20 bis 90° C; und
f) Vermahlen des so erhaltenen Produkts auf eine mittlere Korngröße von 10 bis 1000 µm.

8. Verfahren nach Anspruch 7, **dadurch gekennzeichnet, dass** das Vermengen und Verdichten durch Verkneten erfolgt.

9. Verfahren nach Anspruch 6, **dadurch gekennzeichnet, dass** es die folgenden Stufen umfasst:
a) Vermischen der pulverförmigen Bestandteile Natrium-Carboxymethylstärkeund Cellulose;
b) Sieben durch ein feines Sieb;
c) Verdichten durch Kompaktieren oder Brikettieren mit Kompaktierwalzen oder einer Exzenterpresse bei Drücken von 20 bis 60 kN, vorzugsweise 30 bis 50 kN;
d) Granulieren bzw. verkleinerndes Homogenisieren der nach c) erhaltenen Briketts oder Schülpen mit Stacheiwalzen oder einem ähnlichen Gerät oder Vermahlen mit einer geeigneten Mühle auf Korngrößen von 10 bis 1000µm; und
e) Vermahlen des so erhaltenen Produkts auf eine mittlere Korngröße von 10 bis 1000 µm.

10. Verwendung des Polysaccharidprodukts nach den Ansprüchen 1 bis 5 als Dispersions- oder Suspensionsstabillsator bei der Herstellung von flüssigen und halbfesten Zubereitungen.

11. Verwendung des Polysaccharidprodukts nach den Ansprüchen 1 bis 5 als Tablettensprengmittel.

## Claims

1. Polysaccharide product consisting of high molecular weight, insoluble potato or maize carboxymethylstarch in the form of sodium salt and microcrystalline cellulose or pulverized cellulose, **characterized in that** the insoluble sodium carboxymethylstarch and the microcrystalline cellulose or pulverized cellulose are brought together and compressed by coprocessing by means of wet or dry aggregation under pressure.

2. Polysaccharide product according to Claim 1, **characterized in that** the polysaccharide product has a higher bulk density compared with the starting powder mixture.

3. Polysaccharide product according to Claim 1 or 2, **characterized in that** it has a residual moisture of less than 10%.

4. Polysaccharide product according to one of Claims 1 to 3, **characterized in that** it comprises 3 to 60% by weight of insoluble sodium carboxymethylstarch and 40 to 97% by weight of microcrystalline or pulverized cellulose.

5. Polysaccharide product according to one of Claims 1 to 4, **characterized in that** it has an average particle size of from 10 to 1 000 µm.

6. Process for the preparation of a polysaccharide product consisting of high molecular weight, insoluble potato or maize carboxymethylstarch and microcrystalline or pulverized cellulose, comprising a coprocessing by wet or dry aggregation under pressure of insoluble sodium carboxymethylstarch with microcrystalline cellulose or pulverized cellulose accompanied by a compression.

7. Process according to Claim 6, **characterized in that** it comprises the following stages
a) suspension and optional compression of wet or undried, nonkeratinized microcrystalline or pulverized cellulose in an aqueous medium to give a-homogeneous dispersion;
b) mixing the suspension obtained in stage a) or paste-like, precompressed masses after cooling below 40°C with sodium carboxymethylstarch;
c) optional predrying;
d) compressive conveyance, preferably pressing, of the precompressed wet-plastic mass obtained in stage b) or c) through a sieve, a perforated disc or an extruder;
e) drying of the aggregated mass obtained in stage d) at a temperature from 20 to 90°C; and
f) grinding of the product obtained in this way to an average particle size of from 10 to 1 000 µm.

8. Process according to Claim 7, **characterized in that** the mixing and compression takes place by kneading.

9. Process according to Claim 6, **characterized in that** it comprises the following stages:
a) mixing the pulverulent constituents sodium carboxymethylstarch and cellulose;
b) sieving through a fine sieve;
c) compression by compaction or briquetting with compacting rolls or an eccentric press at pressures of from 20 to 60 kN, preferably 30 to 50 kN;
d) granulation and/or comminuting homogenization of the briquettes or flakes obtained by c) with toothed rolls or a similar device or grinding with a suitable mill to particle sizes of from 10 to 1000 µm and
e) grinding of the product obtained in this way to an average particle size of from 10 to 1000 µm.

10. Use of the polysaccharide product according to Claims 1 to 5 as dispersion or suspension stabilizer in the preparation of liquid and semisolid preparations.

11. Use of the polysaccharide product according to Claims 1 to 5 as tablet disintegrants.

## Revendications

1. Produit de polysaccharides, se composant de carboxyméthylamidon de pommes de terre ou de maïs insoluble, à poids moléculaire élevé, sous la forme du sel de sodium et de cellulose microcristalline ou de cellulose pulvérisée, **caractérisé en ce que** le carboxyméthylamidon de sodium insoluble et la cellulose microcristalline ou la cellulose pulvérisée sont combinés et comprimés par traitement en parallèle sous pression par l'intermédiaire d'une agrégation par voie humide ou par voie sèche.

2. Produit de polysaccharides selon la revendication 1, **caractérisé en ce que** le produit de polysaccharides possède une densité en vrac plus élevée par rapport au mélange de poudres de départ.

3. Produit de polysaccharides selon la revendication 1 ou 2, **caractérisé en ce qu'**il possède une humidité résiduelle de moins de 10%.

4. Produit de polysaccharides selon l'une quelconque des revendications 1 à 3, **caractérisé en ce qu'**il contient de 3 à 60% en poids de carboxyméthylamidon de sodium insoluble et de 40 à 97% en poids de cellulose microcristalline ou de cellulose pulvérisée.

5. Produit de polysaccharides selon l'une quelconque des revendications 1 à 4, **caractérisé en ce qu'**il possède une granulométrie moyenne de 10 à 1000 µn.

6. Procédé de fabrication d'un produit de polysaccharides se composant de carboxyméthylamidon de pommes de terre ou de maïs insoluble, à poids moléculaire élevé, et de cellulose microcristalline ou pulvérisée, incluant un traitement en parallèle sous pression, par agrégation par voie humide ou par voie sèche, de carboxyméthylamidon de sodium insoluble avec de la cellulose microcristalline ou de la cellulose pulvérisée, conjointement à une compression.

7. Procédé selon la revendication 6, **caractérisé en ce qu'**il comprend les étapes suivantes
a) la suspension et, le cas échéant, la compaction de cellulose microcristalline ou pulvérisée humide ou non séchée non crabotée, dans un milieu aqueux pour former une dispersion homogène ;
b) le mélange de la suspension obtenue dans l'étape a) ou de masses pâteuses, soumises à une compaction préalable, après refroidissement en dessous de 40°C avec du carboxyméthylamidon de sodium ;
c) le cas échéant, un séchage préalable ;
d) le passage compactant, de préférence la compression de la masse plastique humide, obtenue dans l'étape b) ou c) et soumise à compaction préalable, à travers un tamis, un disque perforé ou une extrudeuse ;
e) le séchage de la masse agrégée obtenue dans l'étape d) à une température de 20 à 90°C; et
f) la mouture du produit ainsi obtenu à une granulométrie moyenne de 10 à 1000 µm.

8. Procédé selon la revendication 7, **caractérisé en ce que** le mélange et la compaction se font par pétrissage.

9. Procédé selon la revendication 6, **caractérisé en ce qu'**il comprend les étapes suivantes:
a) le mélange des constituants pulvérulents carboxyméthylamidon de sodium et cellulose ;
b) le tamisage à travers un tamis fin ;
c) la compression par compaction ou briquetage à l'aide de rouleaux de compaction ou d'une presse à excentrique, à des pressions de 20 à 60 kN, de préférence de 30 à 50 kN ;
d) la granulation ou l'homogénéisation avec fragmentation de la briquette ou des écailles, obtenues selon c), par des rouleaux à dents ou par un appareil similaire ou la mouture à l'aide d'un moulin approprié à des granulométries de 10 à 1000 µm ; et
e) la mouture du produit ainsi obtenu à une granulométrie moyenne de 10 à 1000 µm.

10. Utilisation du produit de polysaccharides selon les revendications 1 à 5, en tant qu'agent stabilisateur de dispersion ou de suspension lors de la fabrication de préparations liquides et semi-solides.

11. Utilisation du produit de polysaccharides selon les revendications 1 à 5 en tant qu'agent de désintégration de tablettes.
